# EUROPEAN PATENT APPLICATION

(11) **EP 2 006 800 A1**
(43) Date of publication of application: **24.12.2008**
(21) Application number: 07740968.8
(22) Date of filing: 04.04.2007
(51) Int. Cl.: G06Q 50/00, A61B 5/00

(54) **MEDICAL INFORMATION PROCESSING DEVICE**

(30) Priority: 06.04.2006 JP 2006105295
(71) Applicant: Konica Minolta Medical & Graphic, Inc., Hino-shi, Tokyo 191-8511 (JP)
(72) Inventor: SASANO, Yasuhiko, Hachioji-shi, Tokyo 192-8505 (JP)
(74) Representative: Gille, Christian
(86) International application number: PCT/JP2007/057532
(87) International publication number: WO 2007/116899

(57) **Abstract**

In a medical information processing apparatus having a display section for displaying a medical image, when examination order information disagrees with accompanying information of the medical image, a user can promptly recognize it. According to an image examining apparatus 3 as the medical information processing apparatus of the present invention, when image data of a medical image is received from a modality 2 as an image generating apparatus, information of an item set in a comparison object item setting file 361 is extracted from the accompanying information of the received image data and the corresponding examination order information received form RIS 1 as an information managing apparatus. The extracted accompanying information is compared to the examination order information for each of the same items. When there is an item causing a disagreement, an inconsistency informing icon 331a is displayed on an image examination list screen 331 and the accompanying information including the item causing the disagreement on the accompanying information list is identifiably displayed with a color different from that of the other accompanying information.

## Description

### Technical Field

The present invention relates to a medical information processing apparatus. The present invention particularly relates to a medical information processing apparatus (hereinafter referred to as an image examining apparatus) for displaying a medical image generated by an image generating apparatus on the basis of examination order information and the accompanying information of the medical image on a display section to perform the confirmation and correction of the medical image and the accompanying information thereof.

### Background Art

In the medical field, the digitization of medical images obtained by the radiographing of patients has been realized, and the following medical image systems have been utilized. That is, the image data of medical images which have been radiographed and digitized by various image generating apparatuses (modalities) such as computed radiography (CR) apparatus, computed tomography (CT) apparatus, and magnetic resonance imaging (MRI) apparatus, is saved in a picture archiving and communication system for medical application (PACS), and is displayed on image displaying apparatuses for diagnoses by doctors, together with the accompanying information thereof such as the patient information of the patients to be examined, examination information (such as the information of the modalities used for the examinations, radiographing region information, radiographing direction information, and radiographing dates), and the like.

A modality performs radiographing in accordance with the examination order information registered in a radiological information system (RIS) or a hospital information system (HIS), and the modality makes the image data of the medical image obtained by the radiographing accompany the patient information and the examination information on the basis of the examination order information as the accompanying information to transmit the image data accompanied by the accompany information to the PACS and the image displaying apparatus.

However, some modalities cannot link the RIS or the HIS with data, and operators sometimes input the patient information and the examination information to modalities newly to make the input information be accompanied by the medical images. In these cases, the case in which the content of examination order information does not agree with the content of the accompanying information of a medical image owing to an operator's inputting error can be caused. Moreover, even when a modality is the one capable of linking data with the RIS or the HIS, when the maker of the modality is a different one from that of the RIS or the HIS, the modality converts the specification of received examination order information into that of the modality so as to conform with the modality, and the examination order information can be changed to erroneous information at the time of the conversion. When the disagreement of the content of examination order information and that of the accompanying information of the medical image is caused in this manner, then adverse effect such as the impossibility of the specification of radiographed medical images is produced.

Accordingly, for example, Patent Document 1 describes a technique in which an integrating server performs the following operations: comparing examination order information with image attribute information, such as the patient identification (ID), examination date, and examination assortment of a medical image; providing the image attribute information with consistent state identifying information indicating the consistent state of both of the examination order information and the image attribute information; storing the image attribute information with the provided consistent state identifying information in an image attribute information storing section; retrieving the image attribute information stored in the image attribute information storing section on the basis of an input retrieval key when a user inputs the retrieval key, such as a patient ID or examination date that has been arbitrarily selected by the user with a maintenance terminal; displaying a list of the retrieved image attribute information; displaying the consistent state of the information of the image attribute information selected by the user in the displayed list; and accepting alteration.

Moreover, Patent Document 2 describes a technique of: storing the accompanying information including inconsistency in an inconsistency folder when the inconsistency arises between the input accompanying information, and the basic information stored in a medical image managing system; displaying a list screen of the accompanying information including the inconsistency at the time of displaying a correction screen; obtaining the patient information and examination information corresponding to the input patient ID and examination ID, respectively, from an RIS to display the obtained patient information and examination information as correction candidate information; further obtaining the basic information corresponding to the input patient ID and examination ID similarly also from a basic information database (DB) to display the obtained basic information as the correction candidate information; and performing the correction of the information of the disagreeing item on the basis of any piece of information selected by an administrator among the pieces of accompanying information, RIS information, and basic information.
[Patent Document 1] Japanese Patent Application Laid-Open Publication No. 2004-272402
[Patent Document 2] Japanese Patent Application Laid-Open Publication No. 2005-149111

### Disclosure of the Invention

### Problem to be Solved by the Invention

The techniques described in Patent Documents 1 and 2 do not concern the apparatus for displaying a medical image generated by a modality to confirm it, but concern the technique for a user, such as an administrator, to manually perform the confirmation of inconsistency and corrections thereof with a terminal dedicated for maintenance or a server. In order that the user may know whether there are any pieces of inconsistent information or not, the user is required to perform retrieval by inputting a patient ID, an examination ID, or the like, or to perform the operation of displaying a correcting screen. However, when it is impossible to confirm whether or not there is any inconsistency between accompanying information and examination order information unless a patient ID or an examination ID is input or unless a correcting screen is displayed in an apparatus chiefly displaying a medical image received from a modality for a diagnosis or confirmation, then the operation is troublesome, and there is the possibility that erroneous information remains as it is.

It is an object of the present invention to enable a user to immediately recognize the case where examination order information and the accompanying information of a medical image do not agree with each other in a medical information processing apparatus equipped with a display section to display the medical image.

### Means for Solving the Problem

According to a first aspect of the present invention, a medical information processing apparatus which is connected to an information managing apparatus for generating examination order information, and to an image generating apparatus for generating a medical image based on the examination order information and for accompanying the generated medical image with accompanying information including patient information and examination information both pertaining to the medical image based on the examination order information, so that the medical information processing apparatus, the information managing apparatus and the image generating apparatus mutually transmit and receive data, the medical information processing apparatus comprises:
a display section to display the medical image received from the image generating apparatus;
a list displaying section to display a list of the accompanying information of the medical image to be displayed in the display section, the medical image having been received from the image generating apparatus;
a comparison section to compare a piece of the examination order information received from the information managing apparatus with a piece of the accompanying information of the medical image generated based on the examination order information in the image generating apparatus, by each of the same items, to judge whether a disagreeing item in which the piece of the examination order information and the piece of the accompanying information disagrees with each other exists or not, wherein
in a case where there is a piece of the accompanying information in which it is judged that the disagreeing item exists as a result of a comparison by the comparison section, the list displaying section identifiably displays that the piece of the accompanying information including the disagreeing item exists when the list is displayed.

Preferably, the list displaying section identifiably displays the piece of the accompanying information which is judged by the comparison section to include the disagreeing item, when the list is displayed.

Preferably, the medical information processing apparatus further comprises a setting section to set an item which is an object to be compared by the comparison section, wherein
the comparison section performs the comparison for the set item among items included in the examination order information and in the accompanying information.

Preferably, the medical information processing apparatus further comprises a disagreeing item displaying section to display the disagreeing item when it is judged that the disagreeing item exists as the result of the comparison by the comparison section.

Preferably, the medical information processing apparatus further comprises a disagreeing item correcting section to correct information of the disagreeing item in the accompanying information to corresponding information of the examination order information when it is judged that the disagreeing item exists as the result of the comparison by the comparison section.

Preferably, the medical information processing apparatus further comprises a batch correcting section to correct information of items set as an object to be compared in the accompanying information to corresponding information of the examination order information all at once when it is judged that disagreeing information exists as the result of the comparison by the comparison section.

Preferably, the medical information processing apparatus further comprises an obtainment section to obtain the examination order information corresponding to the accompanying information from the information managing apparatus.

### Effect of the Invention

According to a first aspect of the present invention, a user can recognize whether there is any accompanying information including an item disagreeing with examination order information or not at the time of confirming a list of the accompanying information of the medical images to be displayed in a medical information processing apparatus chiefly for displaying a medical image received from an image generating apparatus. Consequently, the user can immediately recognize the existence of the accompanying information disagreeing with the examination order information without confirming whether there is any disagreeing accompanying information or not, inputting a patient ID and an examination ID for performing a correction, and displaying a correcting screen.

Moreover, by adopting the configuration of identifiably displaying the accompanying information judged to have a disagreeing item by a comparison section when a list displaying section performs the list display of the accompanying information of medical images, a user can immediately recognize which accompanying information disagrees with examination order information in a list of the accompanying information of the medical images to be displayed.

Moreover, by adopting the configuration which is equipped with a setting section to set an item of an object of comparison by a comparison section and which performs the comparison of the item of examination order information with the item set by the setting section among the items included in accompanying information by the comparison section, it becomes possible to compare only the item desired by a user among the items included in the accompanying information with the corresponding item of the examination order information.

Moreover, by adopting the configuration of identifiably displaying which item in the accompanying information disagrees with that of examination order information, the accompanying information disagreeing with the examination order information, a user can immediately specify the disagreeing item.

Moreover, by adopting the configuration equipped with a disagreeing item correcting section to correct the information of the disagreeing item in accompanying information to the corresponding information in examination order information when it is judged that there is a disagreeing item as a result of comparison by a comparison section, it becomes possible to easily correct the item disagreeing with that of the examination order information by the examination order information.

Moreover, by adopting the configuration equipped with a batch correcting section to correct the information of the items of the accompanying information set as the objects to be compared to the corresponding information of examination order information in a batch when it is judged that there is disagreeing information as a result of a comparison by a comparison section, it becomes possible to easily perform the correction in a batch when there is a plurality of items disagreeing with those of the examination order information.

Moreover, by adopting the configuration further equipped with an obtainment section to obtain the examination order information corresponding to accompanying information from an information managing apparatus, it becomes possible to receive the examination order information to perform the confirmation and correction of the accompanying information when no examination order information is received.

### Brief Description of the Drawings

[FIG. 1] This is a diagram showing an example of the whole configuration of a medical image system of an embodiment of the present invention.
[FIG 2] This is a block diagram showing the functional configuration of the image examining apparatus shown in FIG. 1.
[FIG 3] This is a diagram showing an example of a data storage of a comparison object item setting file shown in FIG. 2.
[FIG 4] This is a diagram showing the recording structure of the accompanying information DB shown in FIG. 2.
[FIG. 5A] This is a flow chart showing the processing and flows of data between each of the apparatuses constituting the medical image system shown in FIG. 1.
[FIG. 5B] This is a flow chart showing the processing and flows of data between each of the apparatuses constituting the medical image system shown in FIG. 1.
[FIG. 6] This is a flow chart showing examination order information/accompanying information matching processing executed by the central processing unit (CPU) shown in FIG. 2.
[FIG. 7A] This is a flow chart showing inconsistency correcting processing executed by the CPU shown in FIG. 2. [FIG. 7B] This is a flow chart showing the inconsistency correcting processing executed by the CPU shown in FIG. 2. [FIG. 8] This is a view showing an example of an image examination list screen to be displayed in the display section shown in FIG. 2.
[FIG. 9] This is a view showing an example of an inconsistency correcting screen to be displayed in the display section shown in FIG. 2.

### Best Mode for Carrying out the Invention

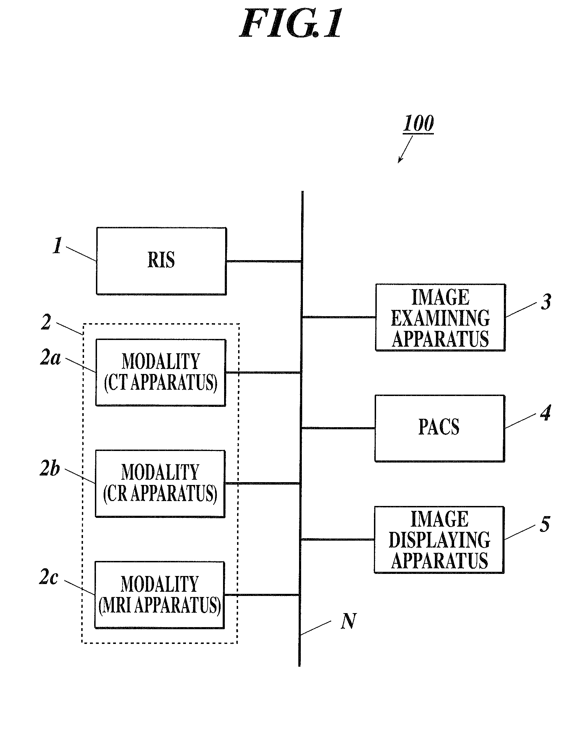

In the following, an embodiment of a medical image system according to the present invention will be described with reference to FIGS. 1-9. However, the present invention is not limited to the shown examples.

First, the configuration of the present embodiment is described.
FIG. 1 shows the system configuration of a medical image system 100.
As shown in FIG. 1, the medical image system 100 is composed of an RIS 1, modalities 2 (2a-2c), an image examining apparatus 3, a PACS 4, and an image displaying apparatus 5, all of which are connected to each other through a communication network N, such as a local area network (LAN) and a wide area network (WAN), in a state of being capable of performing the transmission and reception of data mutually.

In the following, each apparatus constituting the medical image system 100 is described.
The RIS 1 is an information managing apparatus to manage the information in a department of radiology. The RIS 1 accepts an operator's registration of examination order information (such as patient information including the items of a patient ID, a full name, the distinction of sex, and the like for identifying the patient of an examination object; examination information including the items of modality information to be used for the examination (radiographing) of a medical image, a radiographing method (manipulation), radiographing region information, and radiographing direction information; and receipt number) to generate the examination order information, and attaches an examination ID which is the identification information of the examination order information, to the generated examination order information to manage the examination order information. The examination order information includes a piece of patient information, and examination information pertaining to one or a plurality of related examinations. For example, when the examination to be performed to a certain patient is only the examination of radiographing the front breast in a lying position by means of a CR apparatus 2b, then the examination information included in the examination order information is a piece. When the examinations to be performed to a certain patient are the examination of radiographing the front breast in the lying position by means of the CR apparatus 2b and the examination of the breast in the lying position by means of a CT apparatus 2a, then the pieces of examination information included in the examination order information are two pieces. The RIS 1 transmits the generated examination order information to the modalities 2 specified by the modality information of the examination order information and to the image examining apparatus 3.

Incidentally, although the examination order information generated by the RIS 1 is supposed to be transmitted to the modalities 2 and the image examining apparatus 3 in the present embodiment, the examination order information may be generated by a reception terminal which performs the issue and reception of the examination order information, and may be transmitted to the modalities 2.

The modalities 2 are image generating apparatuses to radiograph a patient and to generate the image data of a medical image. Although the medical image system 100 is configured to be equipped with the CT apparatus 2a, the CR apparatus 2b, and an MRI apparatus 2c as the modalities 2 in the present embodiment, modalities to radiograph various kinds of medical images, such as an endoscope apparatus and an ultrasonic diagnostic apparatus, can be applied.

The modalities 2 radiograph a patient in accordance with the examination order information generated by the RIS 1 to generate the image data of the medical image. Then, the modalities 2 make the generated image data of the medical image be accompanied by the examination ID of the examination order information corresponding to the medical image received from the RIS 1, and by the information including the patient information, the examination information and the receipt number for specifying the medical image, among the examination order information as accompanying information, and transmit the image data and the accompanying information to the image examining apparatus 3.

The image data and the accompanying information thereof to be transmitted from the modalities 2 to the image examining apparatus 3 are transmitted as, for example, a digital imaging and communication in medicine (DICOM) file including an image part and a header part. Image data is written into the image part, and the accompanying information (such as the examination ID, the patient information, the examination information, and the receipt number) pertaining to the image data is written into the header part, respectively.

The image examining apparatus 3 is a medical information processing apparatus to receive the examination order information transmitted from the RIS 1, and to receive the image data of a medical image generated by the modalities 2 to display the received medical image and accompanying information in a display section 33 for enabling an image examiner who is the user, to perform the confirmation and correction of the medical image and the accompanying information thereof through an input section 32.

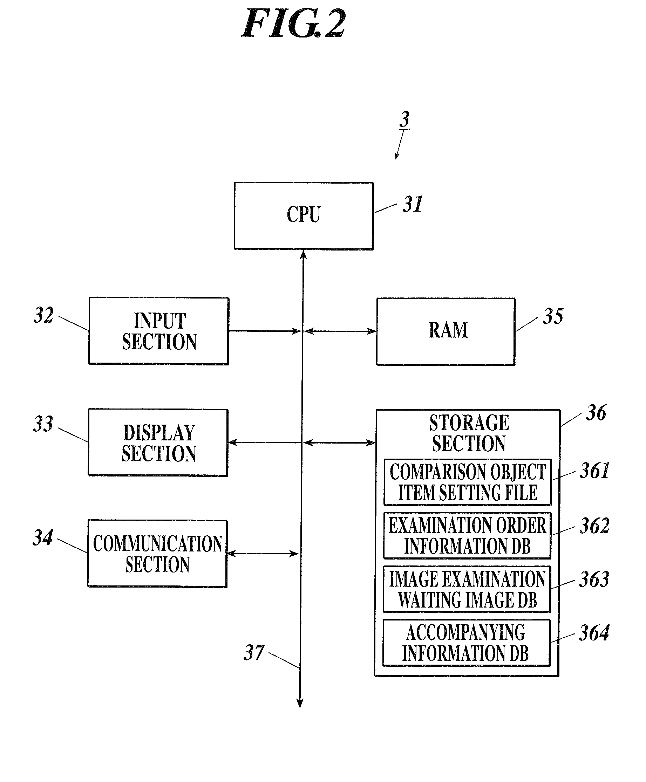

FIG. 2 shows the internal configuration of the image examining apparatus 3.
As shown in FIG. 2, the image examining apparatus 3 is composed of a CPU 31, the input section 32, the display section 33, a communication section 34, a random access memory (RAM) 35, a storage section 36, and the like, and each section is connected to each other through a bus 37.

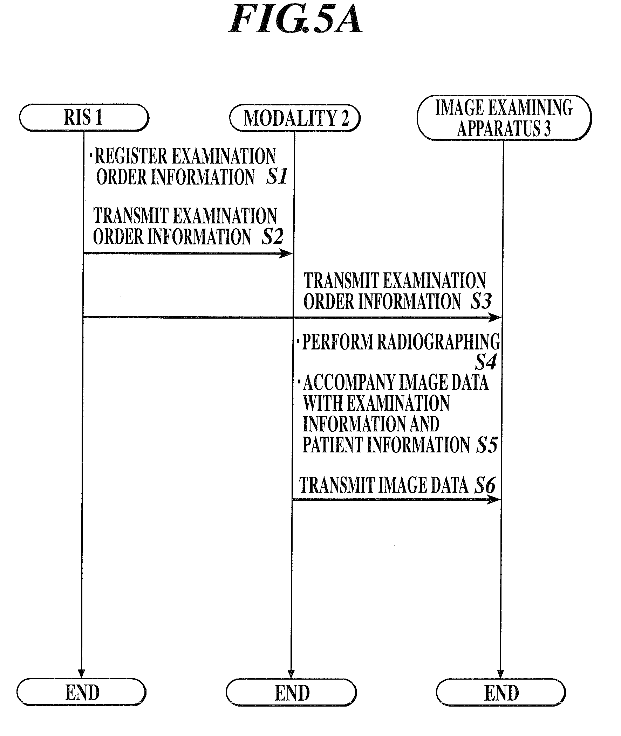

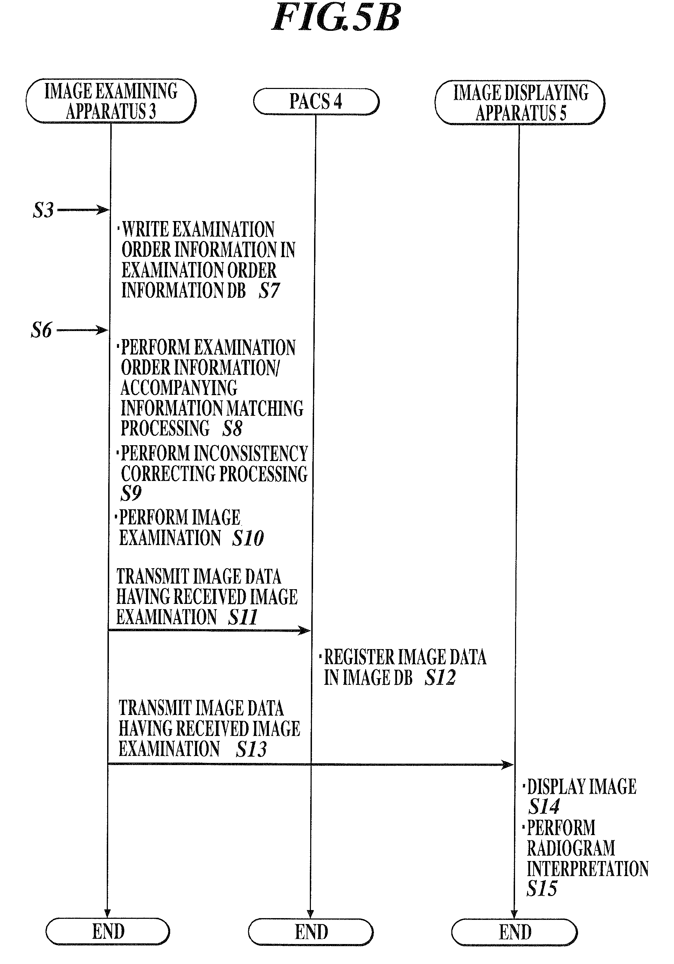

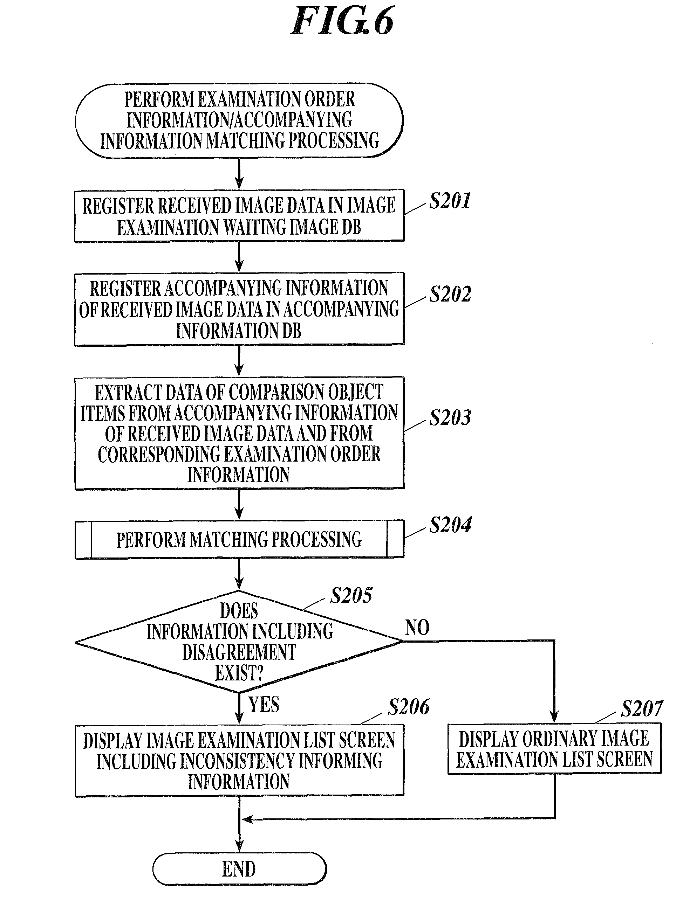

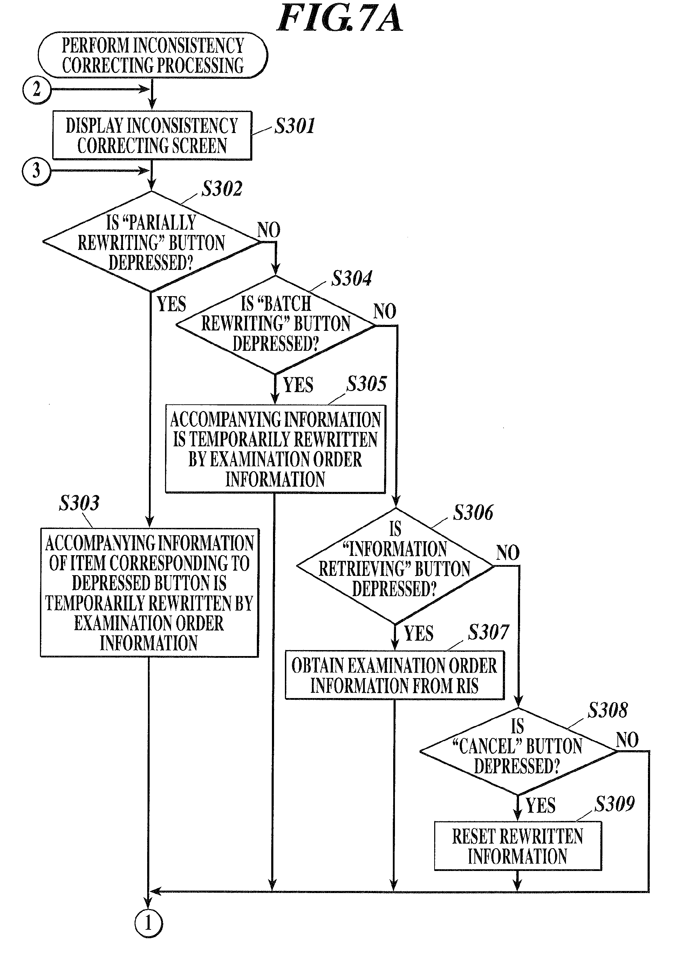

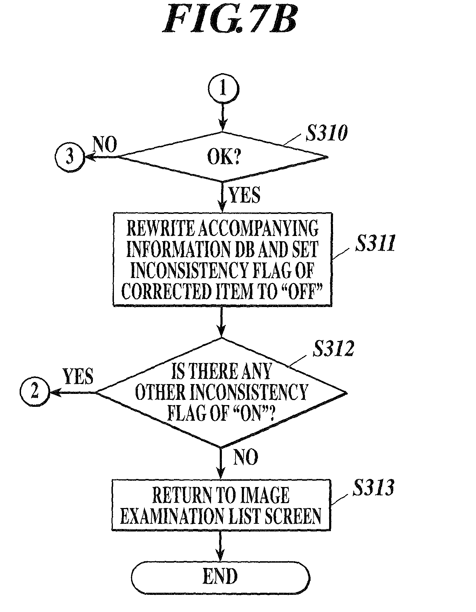

The CPU 31 reads various programs, such as a system program and processing programs, which are stored in the storage section 36, and expands the read programs in the RAM 35. The CPU 31 executes various kinds of processing including the processing on the side of the image examining apparatus 3, which processing is shown in FIGS. 5A and 5B, the examination order information/accompanying information matching processing shown in FIG. 6, and the inconsistency correcting processing shown in FIGS. 7A and 7B, in accordance with the expanded programs.

The input section 32 is composed of a keyboard including cursor keys, numeral inputting keys, various function keys, and the like, and a pointing device, such as a mouse. The input section 32 outputs a depression signal of a key that has received a depression operation with the keyboard, and an operation signal of the mouse to the CPU 31 as input signals.

The display section 33 is composed of a monitor, such as a cathode ray tube (CRT) or a liquid crystal display (LCD), and displays various screens in accordance with the instructions of display signals input from the CPU 31.

The communication section 34 is composed of a network interface and the like, and performs the transmission and reception of data with external equipment connected with the communication network N.

The RAM 35 forms a work area to temporarily store various programs read from the storage section 36, which programs can be executed by the CPU 31, input or output data, parameters, and the like, in various kinds of processing that are executed and controlled by the CPU 31.

The storage section 36 is composed of a hard disk drive (HDD), a semiconductor nonvolatile memory, or the like. The storage section 36 stores various programs including the system program, the examination order information/accompanying information matching processing program, and the inconsistency correcting processing program, which are executed by the CPU 31.

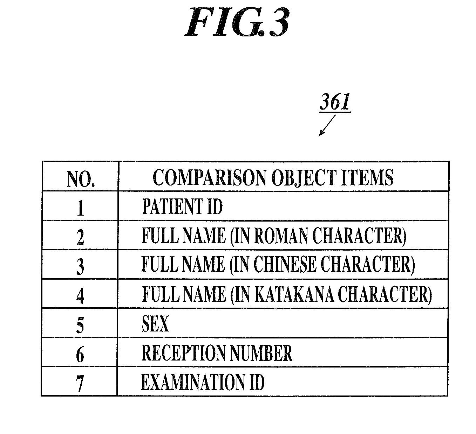

Moreover, the storage section 36 stores a comparison object item setting file 361. The comparison object item setting file 361 is a file to store the items which are to be the objects at the time of comparing the accompanying information of image data with examination order information in the examination order information/accompanying information matching processing shown in FIG. 6. FIG. 3 shows an example of data storage of the comparison object item setting file 361.

Incidentally, the comparison object items in the examination order information/accompanying information matching processing can be arbitrarily set by an image examiner through the input section 32 and comparison object item setting screen (not shown) constituting a setting section. That is, when a comparison object item is set on the not-shown comparison object item setting screen displayed in the display section 33 and the comparison object item is input with the input section 32, the input set content is written in the comparison object item setting file 361, and is set as a comparison object item in matching processing.

Moreover, the storage section 36 includes an examination order information data base (DB) 362 to store the examination order information received from the RIS 1, an image examination waiting image DB 363 to store the image data received from the modalities 2, and an accompanying information DB 364 to store the accompanying information of the image data received from the modalities 2.

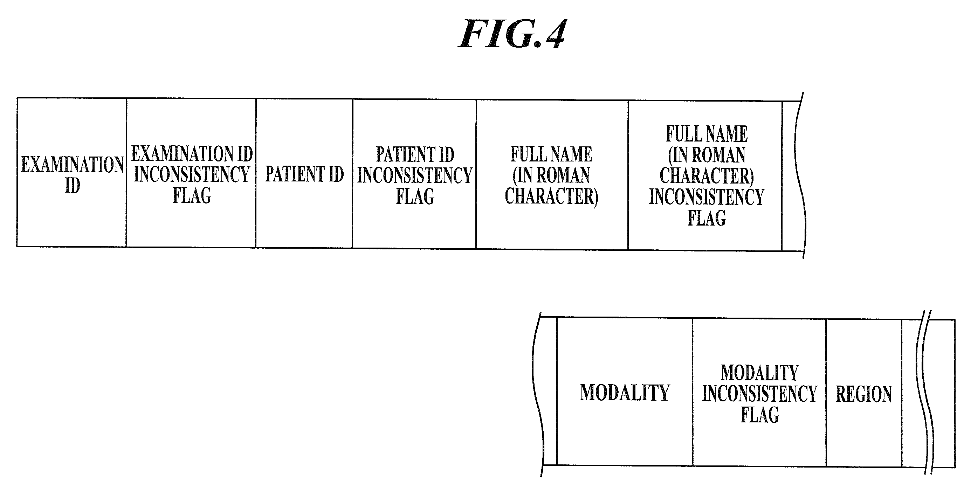

FIG. 4 shows the recording structure of the accompanying information DB 364. As shown in FIG. 4, the accompanying information DB 364 includes the items for storing the information included in accompanying information (such as an "examination ID" item, a "patient ID" item, and so forth); the items of inconsistency flags (such as an "examination ID inconsistency flag" item, a "patient ID inconsistency flag" item, and so forth) for setting an inconsistency flag to an item that disagrees with that of the examination order information when the disagreement (inconsistency) arises as a result of a matching operation, the inconsistency flag items corresponding to each of the items; and the like.

The PACS 4 includes an image DB, and saves the image data of a medical image and the accompanying information thereof that are transmitted from the modalities 2 in the image DB. Moreover, the PACS 4 reads image data from the image DB in response to a request from the image displaying apparatus 5, and distributes the read image data to the image displaying apparatus 5 of the request source.

The image displaying apparatus 5 is a terminal apparatus, such as a personal computer (PC), installed for the purpose of enabling a doctor to perform a radiogram interpretation and a diagnosis by displaying the image data of a medical image distributed from the image examining apparatus 3 or the PACS 4.

Next, the operation of the medical image system 100 is described.
FIGS. 5A and 5B are flow charts showing the processing and flows of data between each apparatus constituting the medical image system 100. In the following, the processing and flows of data between each apparatus constituting the medical image system 100 are described with reference to FIGS. 5A and 5B.

First, examination order information is registered to be generated in the RIS 1 (Step S1), and the generated examination order information is transmitted to one of the modalities 2, the one being specified as the modality to be used for radiographing in the examination order information, and to the image examining apparatus 3 (Steps S2 and S3).

When the modality 2 receives the examination order information from the RIS 1, the modality 2 performs the radiographing of a radiographing region of a patient on the basis of the received examination order information, and the image data of a medical image is generated (Step S4). An examination ID; patient information; examination information pertaining to the image data; a receipt number; and the like, are written in the header part of the generated image data as accompanying information on the basis of the examination order information (Step S5), and the image data accompanied by the accompanying information is transmitted to the image examining apparatus 3 (Step S6).

When the image examining apparatus 3 receives the examination order information transmitted from the RIS 1, the image examining apparatus 3 writes the received examination order information in the examination order information DB 362 (Step S7).

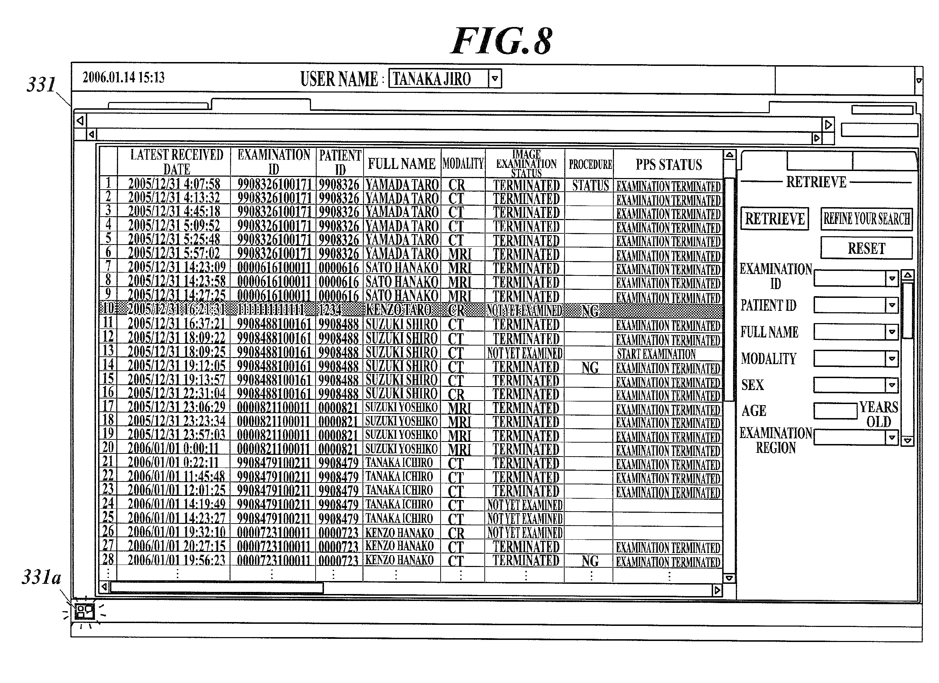

Moreover, when the image examining apparatus 3 receives the image data from the modality 2, the examination order information/accompanying information matching processing shown in FIG. 6 is performed, and an image examination list screen 331 shown in FIG. 8 is displayed in the display section 33 (Step S8). When an inconsistency informing icon 331a is depressed on the image examination list screen 331 through the input section 32, the inconsistency correcting processing shown in FIGS. 7A and 7B is executed (Step S9). Moreover, when a medical image which is to be an image examination object is selected on the image examination list screen 331 through the input section 32, an image examination screen (not shown) including the display of the selected medical image is displayed on the display section 33, and the image examination by the image examiner is performed (Step S10). The image examination means the procedure in which an image examiner refers to an image examination screen to confirm the image quality and position of a medical image and performs the adjustments of the density, contrast, and the like, of an image on the image examination screen through the input section 32 when necessary.

The image data (including the accompanying information) that has received the image examination is transmitted to the PACS 4 (Step S11), and is saved in the image DB (Step S12). Alternatively, the image data that has received the image examination is transmitted to the image displaying apparatus 5 (Step S13), and is displayed on the display screen (Step S14) to be supplied to a doctor's radiogram interpretation diagnosis (Step S15).

In the following, the aforesaid examination order information/accompanying information matching processing and the inconsistency correcting processing are described in detail.
FIG. 6 is a flow chart showing the examination order information/accompanying information matching processing. The processing is the one executed at the time of the reception of the image data of a medical image from one of the modalities 2 with the communication section 34, and is realized by the software processing by the cooperation of the CPU 31 and the examination order information/accompanying information matching processing program. A comparison section and a list displaying section are realized by the execution of the processing.
In the following, the examination order information/accompanying information matching processing is described with reference to FIG. 6.

When the communication section 34 receives the image data of a medical image transmitted from one of the modalities 2, the received image data is registered in the image examination waiting image DB 363 (Step S201), and the accompanying information of the received image data is registered in the accompanying information DB 364 (Step S202). Next, the comparison object item setting file 361 is referred to, and the information of the comparison object items set by the comparison object item setting file 361 among the accompanying information of the received image data is extracted from the accompanying information DB 364, to be read in the work area of the RAM 35. Moreover, the information of the comparison object items among the examination order information having the same examination IDs as those of the accompanying information and corresponding to the accompanying information is extracted from the examination order information DB 362, and the information to be compared is correspondingly read in the work area of the RAM 35 (Step S203).

When the information of the comparison object items is extracted, matching processing is performed. That is, each of the same items of the extracted accompanying information and the examination order information is compared with each other, and whether both of them agree with each other or not is judged. When both pieces of the information are judged not to agree with each other, ON is set in the inconsistency flag of the corresponding item of the accompanying information DB 364 (Step S204).

When the comparison of each of the comparison object items has been completed, it is judged whether the accompanying information including the items having the inconsistency flags in which ON is set exists or not, by searching the accompanying information DB 364. When it is judged that the accompanying information including the items having the inconsistency flags in which ON is set exists (Step S205; YES), then the image examination list screen 331 including inconsistency informing information indicating the existence of the accompanying information including disagreement (inconsistency) with the examination order information is displayed in the display section 33 (Step S206), and the present processing is terminated.

FIG. 8 shows an example of the image examination list screen 331. The image examination list screen 331 is a screen to perform a list display of the content of the accompanying information registered in the accompanying information DB 364. That is, the image examination list screen 331 is a screen to perform the list display of the accompanying information of the medical images to be displayed for image examinations. As the inconsistency informing information, specifically, the accompanying information of the record including inconsistency flags in the accompanying information DB 364 is subjected to the list display identifiably in a color different from those of the accompanying information of the other records. Moreover, the inconsistency informing icon 331a indicating that the accompanying information in which the inconsistency to the examination order information has arisen exists in the list is displayed to be turned on and off in a left below part of the image examination list screen 331.

On the other hand, when it is judged that no information including the set inconsistency flags exists in the accompanying information DB 364 (Step S205; NO), then the ordinary image examination list screen 331 including no inconsistency informing information is displayed in the display section 33 (Step S207), and the present processing is terminated.

As described above, it is identifiably displayed whether the accompanying information including the inconsistency with the examination order information exists or not, by the color of the accompanying information on the list and the existence of the turning on and off of the inconsistency informing icon 331a on the image examination list screen 331.

When the inconsistency informing icon 331a is depressed on the image examination list screen 331, the inconsistency correcting processing is executed.
FIGS. 7A and 7B are flow charts showing the inconsistency correcting processing. The processing is the processing to be realized by the software processing by the cooperation of the CPU 31 and the inconsistency correcting processing program when the inconsistency informing icon 331a on the image examination list screen 331 is depressed, and a disagreeing item displaying section, a disagreeing item correcting section, a batch correcting section, and an obtainment section are realized by the execution of the processing. In the following, the inconsistency correcting processing is described with reference to FIGS. 7A and 7B.

When the inconsistency informing icon 331a is depressed, an inconsistency correcting screen 332 is displayed in the display section 33 (Step S301).

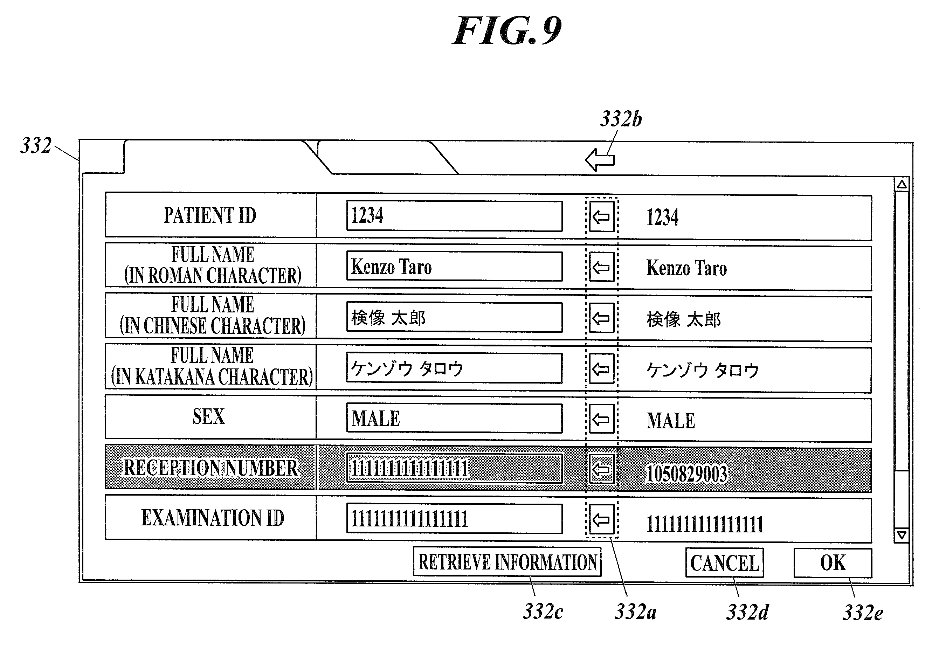

FIG. 9 shows an example of the inconsistency correcting screen 332. As shown in FIG. 9, the inconsistency correcting screen 332 displays the information of each item of the accompanying information including the inconsistency flags, the information being set in the comparison object item setting file 361, together with the corresponding information of the corresponding examination order information. The accompanying information is displayed on the left side of the screen, and the examination order information is displayed on the right side of the screen, respectively. Moreover, the information of the items in which the inconsistency flags are set is identifiably displayed by a color different from those of the other items. FIG. 9 shows the occurrence of the inconsistency between the "receipt number" included in the accompanying information and the "receipt number" included in the examination order information. "Partially rewriting" buttons 332a, in which arrows pointing from pieces of examination order information toward pieces of accompanying information are displayed, are displayed between the accompanying information and examination order information of each item. Moreover, a "batch rewriting" button 332b is displayed at an upper part of the screen. Furthermore, an "information retrieving" button 332c, a "cancel" button 332d, an "OK" button 332e, and the like, are displayed at a lower part of the screen. The "information retrieving" button 332c is a button for obtaining the examination order information corresponding to the displayed accompanying information, and is effective, for example, in the case where the examination order information transmitted from the RIS 1 at Step S3 in FIG. 5A is not received owing to a trouble, a jam, or the like, of communication. Incidentally, the accompanying information and examination order information displayed on the inconsistency correcting screen 332 are temporarily stored in the work area of the RAM 35 in the state of being associated with each other by each item of a comparison object.

When one of the "partially rewriting" buttons 332a is depressed on the inconsistency correcting screen 332 through the input section 32 (Step S302; YES), the accompanying information of the item corresponding to the depressed "partially rewriting" button 332a in the RAM 35 is rewritten to the information of the examination order information (Step S303). When no "partially rewriting" buttons 332a are depressed (Step S302; NO) and the "batch rewriting" button 332b is depressed (Step S304; YES), then the accompanying information temporarily stored in the RAM 35 is rewritten to the examination order information in a batch (Step S305). When the "batch rewriting" button 332b is not depressed (Step S304; NO) and the "information retrieving" button 332c is depressed (Step S306; YES), then the data of the accompanying information displayed on the screen is transmitted to the RIS 1 through the communication section 34, and the obtainment of the examination order information corresponding to the accompanying information from the RIS 1 is performed (Step S307). When the "information retrieving" button 332c is not depressed (Step S306; NO) and the "cancel" button 332d is depressed (Step S308; YES), then the information in the RAM 35 that has been rewritten on the screen is turned back to the state before it was rewritten (Step S309). Incidentally, the accompanying information temporarily stored in the RAM 35 may be rewritten by writing the correct information to the display field of the accompanying information on the screen through the input section 32.

When the "OK" button 332e is depressed after the correction of the information in which inconsistency has arisen (Step S310; YES), then the content of the items of the corresponding accompanying information in the accompanying information DB 364 is written by the content corrected on the inconsistency correcting screen 332, that is, by the accompanying information stored in the RAM 35, and the inconsistency flag corresponding to the corrected information is set to OFF (Step S311). Then, the accompanying information including the inconsistency flags of ON is retrieved from the accompanying information DB 364. When the accompanying information including the inconsistency flags of ON exists (Step S312; YES), then the processing returns to Step S301, and the inconsistency correcting screen 332 of the accompanying information in which the inconsistency has arisen is displayed. When it is judged that the accompanying information including the inconsistency flags of ON does not exist (Step S312; NO), then the display of the display section 33 returns to the image examination list screen 331 (Step S313), and the present processing is terminated.

As described above, according to the image examining apparatus 3, when the image data of a medical image is received from the modalities 2, then the information of the items set in the comparison object item setting file 361 is extracted from the accompanying information of the received image data and the corresponding examination order information received from the RIS 1, and each of the same items of the extracted accompanying information and the examination order information is compared with each other. When there are any disagreeing items, the inconsistency informing icon 331a is displayed on the image examination list screen 331, and the accompanying information including the disagreeing items on the list of the accompanying information is identifiably displayed in a color different from those of the other pieces of accompanying information.

Consequently, an image examiner can recognize whether or not the accompanying information including the items disagreeing with those of the examination order information on the image examination list screen 331 displaying a list of the medical images which are waiting for image examinations, in the image examining apparatus 3 which displays the medical images received from the modalities 2 chiefly for confirmation. Thus, the image examiner can immediately recognize the existence of the accompanying information disagreeing with the examination order information without inputting a patient ID and an examination ID or displaying a correcting screen for confirming whether disagreeing information exists or not, and for correcting the disagreeing information.

Moreover, the inconsistency correcting screen 332 displayed by a depression of the inconsistency informing icon 331a on the image examination list screen 331 identifiably displays which items of accompanying information disagree with those of the examination order information. Thus, an image examiner can immediately specify the disagreeing items to correct them. Since the image examiner can rewrite the information of the items of the accompanying information to the information of the corresponding items of the examination order information only by depressing the "partially rewriting" buttons 332a corresponding to the disagreeing items at the time of the correction, the usability at the time of the correction can be improved, and correction can be easily performed. Moreover, since all the pieces of information of the comparison object items displayed on the inconsistency correcting screen 332 are rewritten to the information of the corresponding items of the examination order information by a depression of the "batch rewriting" button 332b, correction can be easily performed in the case of the existence of a plurality of disagreeing items. Furthermore, since the examination order information can be obtained from the RIS 1 by a depression of the "information retrieving" button 332c, the confirmation and correction of accompanying information can be performed even when, for example, no examination order information is received owing to a defect of communication, or the like.

Incidentally, the content described in the embodiment is a preferred example of the medical image system 100 according to the present invention, and the present invention is not limited to the content of the embodiment.

For example, although the medical image system 100 is configured to be equipped with the RIS 1, the modalities 2, the image examining apparatus 3, the PACS 4, and the image displaying apparatus 5, the medical image system 100 may be equipped with a printer or the like connected thereto and may transmit the image data that has received an image examination to the printer or the like after the completion of the image examination, to output a medical image from the printer or the like.

In addition, also the constructional details and operational details of each apparatus constituting the medical image system 100 can be suitably altered without departing from the sprit and scope of the present invention.

The entire disclosure of Japanese Patent Application No. 2006-105295 filed on April 6, 2006, including description, claims, drawings, and abstract are incorporated herein by reference in its entirety.

### Industrial Applicability

The present invention is usable in the field of medicine, and can be applied to a medical information processing apparatus to perform confirmation and correction of medical images generated by an image generating apparatus, and of accompanying information of the generated medical images.

### Explanation of Reference Numerals

- 100: medical image system
- 1: RIS
- 2: modalities
- 2a: CT apparatus
- 2b: CR apparatus
- 2c: MRI apparatus
- 3: image examining apparatus
- 4: PACS
- 5: image displaying apparatus
- 31: CPU
- 32: input section
- 33: display section
- 331: image examination list screen
- 332: inconsistency correcting screen
- 34: communication section
- 35: RAM
- 36: storage section
- 361: comparison object item setting file
- 362: examination order information DB
- 363: image examination waiting image DB
- 364: accompanying information DB
- 37: bus
- N: communication network

## Claims

1. A medical information processing apparatus which is connected to an information managing apparatus for generating examination order information, and to an image generating apparatus for generating a medical image based on the examination order information and for accompanying the generated medical image with accompanying information including patient information and examination information both pertaining to the medical image based on the examination order information, so that the medical information processing apparatus, the information managing apparatus and the image generating apparatus mutually transmit and receive data, the medical information processing apparatus comprising:
a display section to display the medical image received from the image generating apparatus;
a list displaying section to display a list of the accompanying information of the medical image to be displayed in the display section, the medical image having been received from the image generating apparatus;
a comparison section to compare a piece of the examination order information received from the information managing apparatus with a piece of the accompanying information of the medical image generated based on the examination order information in the image generating apparatus, by each of the same items, to judge whether a disagreeing item in which the piece of the examination order information and the piece of the accompanying information disagree with each other exists or not, wherein
in a case where there is a piece of the accompanying information in which it is judged that the disagreeing item exists as a result of a comparison by the comparison section, the list displaying section identifiably displays that the piece of the accompanying information including the disagreeing item exists when the list is displayed.

2. The medical information processing apparatus of claim 1, wherein
the list displaying section identifiably displays the piece of the accompanying information which is judged by the comparison section to include the disagreeing item, when the list is displayed.

3. The medical information processing apparatus of claim 1, further comprising a setting section to set an item which is an object to be compared by the comparison section, wherein
the comparison section performs the comparison for the set item among items included in the examination order information and in the accompanying information.

4. The medical information processing apparatus of claim 1, further comprising a disagreeing item displaying section to display the disagreeing item when it is judged that the disagreeing item exists as the result of the comparison by the comparison section.

5. The medical information processing apparatus of claim 1, further comprising a disagreeing item correcting section to correct information of the disagreeing item in the accompanying information to corresponding information of the examination order information when it is judged that the disagreeing item exists as the result of the comparison by the comparison section.

6. The medical information processing apparatus of claim 1, further comprising a batch correcting section to correct information of items set as an object to be compared in the accompanying information to corresponding information of the examination order information all at once when it is judged that disagreeing information exists as the result of the comparison by the comparison section.

7. The medical information processing apparatus of claim 1, further comprising an obtainment section to obtain the examination order information corresponding to the accompanying information from the information managing apparatus.
